# EUROPÄISCHE PATENTSCHRIFT

(11) **EP 1 134 267 B1**
(45) Veröffentlichungstag und Bekanntmachung des Hinweises auf die Patenterteilung: **02.10.2002**
(21) Anmeldenummer: 01104900.4
(22) Anmeldetag: 28.02.2001
(51) Int. Cl.: C09J 5/06, C07C 311/17, C08K 5/435

(54) **Weichmacher für Kunststoffe**
Plasticisers for plastics
Plastifiants pour plastiques

(30) Priorität: 15.03.2000 DE 10012618
(43) Veröffentlichungstag der Anmeldung: 19.09.2001
(73) Patentinhaber: Dr. Th. Böhme KG Chem. Fabrik GmbH & Co., 82538 Geretsried (DE)
(72) Erfinder: Balbach, Günter, Dr., 82515 Wolfratshausen (DE); Schönmann, Gertrud, Dr., 82166 Gräfelfing (DE); Ostermann, Jürgen, 82515 Wolfratshausen (DE)
(74) Vertreter: Störle, Christian, Dr.

(56) Entgegenhaltungen:
- EP-A- 0 553 651
- US-A- 3 475 353

## Beschreibung

Die Erfindung bezieht sich auf Verbindungen, die als Weichmacher für polare Kunststoffe geeignet sind, ein Verfahren zu deren Herstellung und deren Verwendung.

Kunststoffweichmacher im engeren Sinn sind Lösungsmittel, die so hoch siedend sind, daß sie bei der Verarbeitung und beim Gebrauch im Kunststoff verbleiben und durch ihre Anwesenheit einen permanenten Quellungszustand des Kunststoffes erzeugen. Dadurch wird die Kohäsion der den Kunststoff aufbauenden Moleküle verringert, was sich makroskopisch in einer größeren Verformbarkeit bei Einwirkung derselben Kraft und in visko-elastischem Verhalten zeigt. Der Kunststoff erscheint somit weicher.

Eine weitere Folge der verminderten Kohäsion ist ein niedrigerer Schmelzpunkt und eine verminderte Schmelzviskosität der Kunststoffe. Diese Veränderungen sind bei der Herstellung und Anwendung von Schmelzklebern von großer Bedeutung.

Die Aufnahme der Weichmacher in Kunststoffe geschieht normalerweise im flüssigen Zustand, bei dem die Weichmachermoleküle beweglich und die zur Anlagerung befähigten Strukturen der Kunststoffe zugänglich sind. Die Verarbeitung von Kunststoffen erfolgt meist im flüssigen Zustand (Schmelze oder Lösung). Die Weichmacher sollten also entweder bei den Verarbeitungstemperaturen flüssig oder in einem gemeinsamen Lösungsmittel mit den Kunststoffen löslich sein. Weiterhin müssen sie mit dem Kunststoff so gut verträglich sein, daß sie während der gesamten Lebensdauer des Kunststoffes nicht "ausschwitzen", also flüssige Filme auf der Oberfläche des Kunststoffes bilden.

Die Verwendung von Arylsulfonamiden als Weichmacher für Kunststoffe ist allgemein bekannt (Taschenbuch der Kunststoff-Additive, München, Wien, 1989, Seiten 418 und 419). Diese enthalten eine Sulfonamidogruppe und sind zur Erzielung der vorgenannten Eigenschaften relativ kleine Moleküle (z. B. N-Butylbenzolsulfonamid). Dies bringt es jedoch mit sich, daß diese Weichmacher flüchtig sind. Die Folge davon sind Emissionen der Arylsulfonamide bei der Verarbeitung der Kunststoffe und eine Verhärtung der Kunststoffe bei der Alterung.

Aus der DE 42 00 768 A1 sind Disulfonamide als flüssige Weichmacher mit niedrigem Dampfdruck für Kunststoffe, wie Polyamide, thermoplastische Polyester, Polyvinylacetate, Polyurethane oder Mischungen davon, bekannt, die eine im Vergleich zu Monosulfonamiden geringere Flüchtigkeit zeigen. Die Herstellungskosten der in der DE 42 00 768 A1 beschriebenen Weichmacher sind jedoch aufgrund der teuren Ausgangsverbindungen sehr hoch.

Der vorliegenden Erfindung liegt somit die Aufgabe zugrunde, Verbindungen bereitzustellen, die als Weichmacher für Kunststoffe eingesetzt werden können, welche die Nachteile der aus dem Stand der Technik bekannten Weichmacher nicht aufweisen.

Erfindungsgemäß wird dies durch eine als Weichmacher für Kunststoffe geeignete Verbindung der Formel (1) erreicht 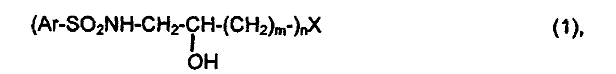 wobei
Ar für einen substituierten oder unsubstituierten Aryl-Rest,
X für einen organischen Rest,
m für eine ganze Zahl von 1 bis 4 und
n für eine ganze Zahl von 2 bis 4 steht.

Wie aus der vorstehenden Formel (1) hervorgeht, können an den organischen Rest X, abhängig von n, 2, 3 oder 4 Reste Ar-SO₂NH-CH₂-CH(OH)-(CH₂)ₘ gebunden sein. Diese können gleich oder verschieden voneinander sein.

Es hat sich als günstig erwiesen, wenn in der erfindungsgemäßen Verbindung m für 1 steht, da die entsprechenden Ausgangsmaterialien, die zur Herstellung der erfindungsgemäßen Verbindung verwendet werden können, besonders leicht und kostengünstig erhältlich sind.

Vorzugsweise ist der Aryl-Rest der erfindungsgemäßen Verbindung ein substituierter oder unsubstituierter Phenyl-Rest, wobei der unsubstituierte Phenyl-Rest vorteilhaft ist, da die ihn enthaltenden Ausgangsverbindungen, die zur Herstellung der erfindungsgemäßen Verbindungen eingesetzt werden, leicht und kostengünstig verfügbar sind. Ferner werden Weichmacher mit besonders guten Eigenschaften erhalten.

Der substituierte Aryl-Rest, insbesondere der substituierte Phenyl-Rest, kann 1 Substituent oder mehrere Substituenten aufweisen, die gleich oder verschieden voneinander sein können. Dieser kann in m-, o- und p-Stellung zur Sulfonamido-Gruppe gebunden sein. Beispiele der Substituenten sind C₁-C₄-Alkyl, wie Methyl, Ethyl, iso- oder n-Propyl, C₁-C₄-Alkoxy, wie Methoxy, Ethoxy, iso- oder n-Propoxy, C₁-C₄-Acyloxy oder Phenoxy. Von diesen ist Methyl besonders günstig, da die entsprechenden methylsubstituierten Aryl-Reste leicht und kostengünstig erhältlich sind und die erfindungsgemäßen Verbindungen sehr gute Eigenschaften als Weichmacher aufweisen.

Wie der vorstehenden Formel (1) entnommen werden kann, weist die erfindungsgemäße Verbindung den organischen Rest X auf, der in ihr als Linker für die Reste Ar-SO₂NH-CH₂-CH(OH)-(CH₂)ₘ fungiert. Als Rest X können organische Verbindungen jeglicher Art eingesetzt werden, die dazu fähig sind, die Reste Ar-SO₂NH-CH₂-CH(OH)-(CH₂)ₘ zu verbinden.

Der Ausdruck "organischer Rest" weist darauf hin, daß es sich dabei um Verbindungen jeglicher Art aus dem Gebiet der organischen Chemie handelt. Bei den gemäß der vorliegenden Erfindung günstigerweise eingesetzten organischen Resten X handelt es sich um Verbindungen des Kohlenstoffs, die weiterhin noch H und/oder O enthalten können. Dabei können C, H und O in unterschiedlichen Mengenverhältnissen vorliegen. Ferner kann der organische Rest X zusätzlich noch Heteroatome, wie N, S, P und/oder Halogene enthalten.

Gemäß der vorstehenden Formel (1) handelt es sich bei dem organischen Rest X um eine zwei-, drei- oder vierwertige Verbindung. Dabei wird im Sinne der vorliegenden Erfindung unter einer "zwei-, drei- oder vierwertigen Verbindung" eine Verbindung verstanden, der im Vergleich zur Stammverbindung zwei, drei oder vier Atome, z. B. Wasserstoffatome, fehlen.

Die in der erfindungsgemäßen Verbindung vorliegenden organischen Reste X sollen keine funktionellen Gruppen aufweisen, die mit den erfindungsgemäßen Verbindungen und/oder dem Kunststoff, in den sie als Weichmacher eingebracht werden können, reagieren können. Des weiteren soll das Molekulargewicht des organischen Restes X so gewählt werden, daß die erfindungsgemäße Verbindung gut in die Kunststoffe eingebaut und somit gut als Weichmacher wirken kann. Solche organischen Reste sind dem Fachmann bekannt, auch weiß er Verfahren und dazu benötigte Materialien, um sie herzustellen.

In einer bevorzugten Ausführungsform der erfindungsgemäßen Verbindung ist der Rest X von einem zwei-, drei oder vierwertigen Alkohol oder einem Polyalkylenglykol abgeleitet. Dabei weist der Ausdruck "abgeleitet" darauf hin, daß die H-Atome der OH-Gruppen dieser Alkohole oder des Polyalkylenglykols durch die Reste Ar-SO₂NH-CH₂-CH(OH)-(CH₂)ₘ substituiert sind.

Vorzugsweise ist der vom Polyalkylenglykol abgeleitete organische Rest X gemäß der Formel (2) definiert.

O-(R¹-O)ₚ (2),

wobei
R¹ für einen zweiwertigen Kohlenwasserstoff-Rest und p für eine Zahl von 1 bis 40, insbesondere 2, 3, 4, 5, und 7 steht.

Unter einem Kohlenwasserstoff-Rest im Sinne der vorliegenden Erfindung werden Verbindungen jeglicher Art verstanden, die aus C- und H-Atomen bestehen. Der Ausdruck zweiwertig weist darauf hin, daß dem in der erfindungsgemäßen Verbindung vorliegenden Rest R¹ im Vergleich zur Stammverbindung zwei H-Atome fehlen.

Beispiele des zweiwertigen Kohlenwasserstoffe-Restes R¹ sind (CH₂)₂, (CH₂)₃, (CH₂)₄ und CH₂CH(CH₃).

Der Rest X gemäß vorstehender Formel (2) kann mehrere wiederkehrende Einheiten R¹-O aufweisen. Diese können gleich oder verschieden voneinander sein.

Beispielsweise kann der Rest X auch eine Verbindung der Formel O[-R^{1'}-O-R^{1"}-O]ₚ sein, wobei R^{1'} und R^{1"} unabhängig voneinander für einen vorstehenden zweiwertigen Kohlenwasserstoff steht. Der Rest X kann auch eine Verbindung der Formel O-(R^{1'}-O)_{p'}-(R^{1"}-O)_{p"} mit p'+p"= p sein, wobei R^{1'} und R^{1"} unabhängig voneinander wie vorstehend definiert sind.

In einer anderen bevorzugten Ausführungsform der erfindungsgemäßen Verbindung ist der von dem zweiwertigen Alkohol abgeleitete organische Rest X gemäß der Formel (3) definiert

O-R-O (3),

wobei R für einen zweiwertigen Kohlenwasserstoff-Rest steht.

Dabei werden unter dem Ausdruck "zweiwertiger Kohlenwasserstoff-Rest" in Verbindung mit Formel (3) diejenigen verstanden, die vorstehend in Verbindung mit Formel (2) angeben sind.

Beispiele der zweiwertigen Kohlenwasserstoff-Reste sind (CH₂)₀ oder (CH₂CH(CH₃))ₒ wobei o jeweils für eine Zahl von 1 bis 40 steht. Vertreter der organischen Reste X gemäß Formel (3) sind von Ethylenglykol, Propylenglykol und 1,6-Hexandiol abgeleitete Verbindungen, in denen die Wasserstoffatome der Hydroxyl-Gruppen fehlen.

In einer weiteren bevorzugten Ausführungsform der erfindungsgemäßen Verbindung steht der von dem drei- oder vierwertigen Alkohol abgeleitete organische Rest X für 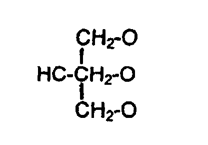 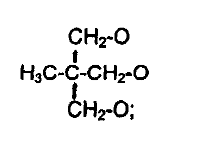 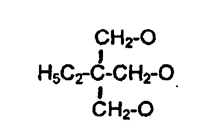 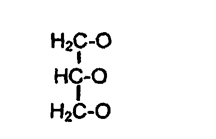 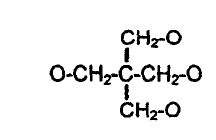 oder

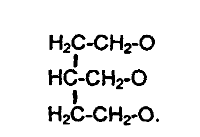

Vorzugsweise kann der Rest X von einem alkoxylierten zwei-, drei- oder vierwertigen Alkohol abgeleitet sein. Dabei handelt es sich um Verbindungen, die durch Umsetzung der vorgenannten zwei-, drei- oder vierwertigen Alkohole mit einer eine Epoxid-Gruppe enthaltenden Verbindung, insbesondere einem Alkylenoxid, wie Ethylenoxid oder Propylenoxid, erhältlich sind.

Das Verhältnis der OH-Gruppen des Alkohols zu den Epoxidgruppen kann dabei in weiten Bereichen variieren. Beispielsweise kann das Verhältnis so gewählt werden, daß nicht alle OH-Gruppen des Alkohols mit einer Epoxid-Gruppe reagieren. Es kann aber auch so sein, daß ein Überschuß von Epoxid-Gruppen, bezogen auf die Anzahl der OH-Gruppen des Alkohols, eingesetzt wird, wobei Polyether gebildet werden können.

Durch die Reaktion der OH-Gruppe mit der Epoxid-Gruppe wird diese gespalten und es entsteht eine neue OH-Gruppe, die zur Bindung mit dem Rest Ar-SO₂NH-CH₂-CH(OH)-(CH₂)ₘ dienen kann.

Günstigerweise kann an einem oder an mehreren der O-Atome des Alkohols ein Rest O(R¹-O)ₚ gebunden sein, wobei R¹ und p wie vorstehend definiert sind. Ein Vertreter davon ist 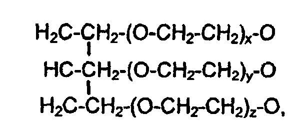 wobei x+y+z 3 bis 7, insbesondere 5, beträgt. Diese Verbindung kann in einfacher Weise durch übliche Ethoxylierung des entsprechenden Alkohols erhalten werden.

Es wurde nun überraschenderweise gefunden, daß die erfindungsgemäßen Verbindungen hervorragende Eigenschaften als Weichmacher, insbesondere für polare Kunststoffe und auch für Polymerschmelzkleber, aufweisen. Dabei besitzen sie eine deutlich geringere Flüchtigkeit als die bisher als Weichmacher eingesetzten Monosulfonamide, so daß die bei den Monosulfonamiden beobachteten unerwünschten Emissionen nicht auftreten. Des weiteren können sie aus kostengünstigeren Rohstoffen als die aus dem Stand der Technik bekannten Disulfonamid-Weichmacher hergestellt werden, d. h., die erfindungsgemäßen Verbindungen sind kostengünstiger als die bekannten Disulfonamide. Ferner werden die erfindungsgemäßen Verbindungen in Kunststoff leichter eingebaut als diese Disulfonamide. Des weiteren sind die erfindungsgemäßen Verbindungen meist flüssig, wodurch sie sehr gut handhabbar sind.

Gegenstand der vorliegenden Erfindung ist femer ein Verfahren zur Herstellung der erfindungsgemäßen Verbindungen, wobei ein Epoxid der Formel (4) 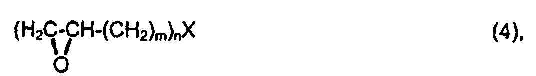 mit einem Arylsulfonamid der Formel (5)

Ar-SO₂NH₂ (5),

wobei Ar, X, m und n wie vorstehend definiert sind, umgesetzt wird. Günstigerweise erfolgt die Umsetzung in einer stöchiometrischen Menge, d. h. pro Epoxid-Gruppe kann ein Arylsulfonamid eingesetzt werden, so daß alle Epoxid-Gruppen mit einem Arylsulfonamid reagieren können.

Epoxide der Formel (4) sind kommerziell erhältlich, wie 1,6-Hexandioldiglycidether, Polypropylenglykoldiglycidether, Trimethylolpropantriglycidether oder Pentaerythrittetraglycidether, oder können in dem Fachmann bekannter Weise hergestellt werden, der die hierzu notwendigen Materialien und Vorrichtungen kennt. Beispielsweise kann Epichlorhydrin mit den vorgenannten zwei-, drei- oder vierwertigen Alkoholen oder den Polyalkylenglykolen in üblicher Weise umgesetzt werden.

Arylsulfonamide der Formel (5) sind ebenfalls kommerziell erhältlich bzw. können in dem Fachmann bekannter Weise hergestellt werden. Ein Beispiel des Arylsulfonamids ist Benzolsulfonamid, das wegen seiner leichten Verfügbarkeit besonders bevorzugt ist.

Die Umsetzung des Epoxids mit dem Arylsulfonamid kann in Reaktionsgefäßen jeglicher Art durchgeführt werden. Ein Lösungsmittel muß bei dieser Reaktion nicht verwendet werden, so daß dadurch hohe Reaktionsgeschwindigkeiten und somit eine sehr schnelle, effektive und kostengünstige Herstellung der erfindungsgemäßen Verbindung erreicht werden kann. Falls es jedoch gewünscht wird, können Lösungsmittel eingesetzt werden. Dabei sind solche günstig, in denen sich die Edukte, d. h. das Epoxid und das Arylsulfonamid, lösen, da dadurch besonders hohe Reaktionsgeschwindigkeiten erzielt werden.

Die erfindungsgemäße Umsetzung wird vorzugsweise bei einer erhöhten Temperatur durchgeführt. Die Temperatur wird dabei günstigerweise so gewählt, daß die Reaktion ausreichend schnell verläuft, aber weder die Edukte noch die Produkte (erfindungsgemäßen Verbindungen) zerstört werden. Dies wird günstigerweise bei einer Temperatur von etwa 80°C bis 140°C erreicht.

Das erfindungsgemäße Verfahren wird üblicherweise solange durchgeführt, bis eine vollständige oder zumindest nahezu vollständige Umsetzung des Epoxids mit dem Arylsulfonamid zur erfindungsgemäßen Verbindung stattgefunden hat. Der Reaktionsverlauf zur Bestimmung der Vervollständigung der Reaktion kann in üblicher Weise erfolgen, beispielsweise durch Probenentnahme aus dem Reaktionsansatz in bestimmten Zeitintervallen und Ermittlung der Menge der Edukte. Die Reaktion ist dann vervollständigt, wenn sich die Menge der Edukte nicht mehr ändert. Es wurde dabei überraschenderweise gefunden, daß das erfindungsgemäße Verfahren nahezu quantitativ abläuft, d. h. nach Vervollständigung der Reaktion hat das Epoxid und das Arylsulfonamid fast vollständig zur erfindungsgemäßen Verbindung reagiert. Dadurch ist es möglich, die erfindungsgemäße Verbindung ohne weitere Reinigung beispielsweise als Weichmacher für polare Kunststoffe einzusetzen.

Sollte eine Reinigung der erfindungsgemäßen Verbindung notwendig sein oder gewünscht werden, so kann diese durch übliche Reinigungsverfahren erfolgen, z. B. Filtration, Extraktion, Destillation und verschiedene chromatographische Verfahren, wie Säulenchromatographie.

Zur Vermeidung der Bildung von unerwünschten Nebenprodukten kann das erfindungsgemäße Verfahren unter Inertgas, z. B. Stickstoff undloder Argon, durchgeführt werden.

In einer bevorzugten Ausführungsform des erfindungsgemäßen Verfahrens können Katalysatoren zur Erhöhung der Reaktionsgeschwindigkeit verwendet werden. Als besonders günstig haben sich dabei basische Katalysatoren herausgestellt, da diese überraschenderweise die Reaktionsgeschwindigkeit besonders stark erhöhen. Beispiele basischer Katalysatoren sind 2,4,6-Tris-(N,N-dimethylaminomethyl)-phenol und 1,5-Diazabicyclo-[4,3,0]-non-5en. Die Katalysatoren können in einer Menge von 0,1 bis 5 Gew.-%, insbesondere etwa 1 Gew.-%, bezogen auf das Epoxid und das Arylsulfonamid, eingesetzt werden. Durch Verwendung dieser Katalysatoren ist die Umsetzung des Epoxids mit dem Arylsulfonamid in 0,5 bis 1 Stunde zumindest nahezu vervollständigt.

Überraschenderweise wurde nun gefunden, daß die erfindungsgemäßen Verbindungen mit dem erfindungsgemäßen Verfahren in besonders schneller, einfacher und kostengünstiger Weise in besonders hohen Ausbeuten hergestellt werden können.

Gegenstand der vorliegenden Erfindung ist femer die Verwendung einer erfindungsgemäßen Verbindung als Weichmacher für Kunststoffe, insbesondere für polare Kunststoffe, wie Polyester, Copolyester, Polyamide, Copolyamide und Polyurethane. Vorzugsweise werden die erfindungsgemäßen Verbindungen für solche Kunststoffe eingesetzt, die als Polymerschmelzkleber verwendet werden. Die Verwendung von Weichmachern für Kunststoffe ist dem Fachmann bekannt. Auch kennt er hierzu notwendige Materialien. Beispielsweise können die erfindungsgemäßen Verbindungen als Weichmacher für Kunststoffe verwendet werden, wie es in der vorgenannten DE 42 00 768 A1 beschrieben ist.

Die erfindungsgemäßen Verbindungen können beispielsweise wie folgt als Weichmacher für Schmelzkleber verwendet werden: Für die Schmelzkleber werden Schmelzkleberpasten unter Verwendung eines üblichen Dispergiermittels hergestellt. Dazu können z. B. etwa 75,9 g Wasser gerührt und etwa 2 g einer handelsüblichen löslichen Stärke eingestreut werden. Es kann gerührt werden, bis die Stärke sich glatt gelöst hat. Diese Lösung kann auf etwa 60°C erhitzt werden. Parallel dazu können etwa 10 g eines Reaktionsproduktes von etwa 1 mol Stearylalkohol mit etwa 4 mol Ethylenoxid aufgeschmolzen werden. Die Schmelze kann der Stärkelösung zugesetzt und unter kräftigem Rühren abgekühlt werden. Es entsteht eine stabile weiße Dispersion. Jetzt können etwa 3 g Polyvinylpyrrolidon mit einem mittleren Molekulargewicht Mᵥ von etwa 38 000 Dalton gelöst in etwa 9 g Wasser unter Rühren zugesetzt werden. Dazu können etwa 2 g eines handelsüblichen Silikonentschäumers zugegeben werden, um, falls es nötig ist, die Schaumbildung zu vermindern. Danach können etwa 24 Gew.-Teile des Dispergiermittels, etwa 41 Gew.-Teile Wasser, etwa 3 Gew.-Teile einer 5%igen Lösung eines üblichen Diurethanverdickers, etwa 3 Gew.-Teile einer 25%igen Lösung eines Na-Salzes eines üblichen Polyacrylsäureverdickers, etwa 35 Gew.-Teile eines handelsüblichen Pulvers eines thermoplastischen Polyesters, etwa 10 Gew.-Teile einer 1%igen Lösung eines üblichen hochmolekularen Polyethylenglykols und etwa 7 Gew.-Teile der erfindungsgemäßen Verbindung vermischt werden, wodurch eine Schmelzkleberpaste erhalten wird, die in üblicher Weise eingesetzt werden kann.

Die Erfindung wird anhand der nachfolgenden Beispiele erläutert.

### Beispiel 1: Herstellung einer erfindungsgemäßen Verbindung aus einem Polypropylenglykoldiglycidether und Benzolsulfonamid

In einem Becherglas wurden 33,1 g (1 mol) eines Polypropylenglykoldiglycidethers mit 6 bis 7 von Polypropylen abgeleiteten Einheiten mit 15,7 g (2 mol) Benzolsulfonamid in Gegenwart von 1 Gew.-% DBN während etwa 30 bis 60 Minuten bei 120 °C umgesetzt. Die Reaktion war exotherm. Es wurde ein bräunliches, zähflüssiges Produkt erhalten, das dem erwarteten Umsetzungsprodukt des vorgenannten Polypropylenglykoldiglycidethers mit Benzolsulfonamid entsprach.

### Beispiel 2: Herstellung einer erfindungsgemäßen Verbindung aus einem Polypropylenglykoldiglycidether und Benzolsulfonamid

In einem Becherglas wurden 24,1 g (1 mol) eines Polypropylenglykoldiglycidethers mit 3 bis 4 von Polypropylenglycol abgeleiteten Einheiten in Gegenwart von 1 Gew.-% DBN als Katalysator bei 100°C während ca. 30 bis 60 Minuten umgesetzt. Die Reaktion war exotherm. Es wurde ein gelbes, flüssiges Produkt erhalten, das dem erwarteten Umsetzungsprodukt des vorgenannten Polypropylenglykoldiglycidethers mit Benzolsulfonamid entsprach.

### Beispiel 3: Herstellung einer erfindungsgemäßen Verbindung durch Umsetzung von Benzolsulfonamid mit einem Epoxid

In üblicher Weise wurde 1 Mol der Verbindung 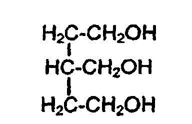 mit 5 Mol Ethylenoxid und nachfolgend mit 3 Mol Epichlorhydrin in üblicher Weise umgesetzt, wodurch ein Produkt mit 3 Epoxid-Gruppen erhalten wurde.

In einem Becherglas wurden 24,5 g (1 mol) dieses Produkts mit 15,7 g (3 mol) Benzolsulfonamid in Gegenwart von 1 Gew.-% DBN als Katalysator bei 120°C während 1 Stunde umgesetzt. Die Reaktion war exotherm. Es wurde ein gelbes, zähflüssiges Produkt erhalten, das dem erwarteten Umsetzungsprodukt von Benzolsulfonamid mit dem vorgenannten Epoxid entsprach.

### Beispiel 4: Herstellung einer erfindungsgemäßen Verbindung aus Pentaerythrittetraglycidether und Benzolsulfonamid

In einem Becherglas wurden 33,3 g (1 mol) Pentaerythrittetraglycidether mit 31,4 g (4 mol) Benzolsulfonamid in Gegenwart von 1 Gew.-% 1,5-Diazabicyclo-[4,3,0]non-5en (DBN) bei einer Temperatur von 100°C während ca. 30 Minuten umgesetzt. Die Reaktion war exotherm. Es wurde ein festes Produkt erhalten, das dem erwarteten Umsetzungsprodukt von Pentaerythrittetraglycidether mit Benzolsulfonamid entsprach.

### Beispiel 5: Plastifizierende Wirkung erfindungsgemäßer Verbindungen

Die plastifizierende Wirkung der'erfindungsgemäßen Verbindungen der Beispiele 1 bis 3 wurde durch Einarbeiten in einen aus wäßriger Dispersion aufgebrachten Schmelzkleber geprüft. Eine Plastifizierung des im Kleber verwendeten Polymers zeigte sich in einer verbesserten Haftung bei niedrigen Verklebungstemperaturen.

Zur Herstellung eines Dispergiermittels wurden 75,9 g Wasser gerührt und 2 g einer handelsüblichen löslichen Stärke eingebracht. Es wurde gerührt bis die Stärke vollständig gelöst war. Diese Lösung wurde auf 60°C erhitzt. Parallel dazu wurden 10 g eines Reaktionsprodukts von 1 mol Stearylalkohol mit 4 mol Ethylenoxid aufgeschmolzen. Die Schmelze wurde der Stärkelösung zugesetzt und unter kräftigem Rühren abgekühlt. Es entstand eine stabile weiße Dispersion. Danach wurden 3 g Polyvinylpyrrolidon mit einem mittleren Molekulargewicht Mᵥ=38 000 Dalton gelöst in 9 g Wasser unter Rühren zugesetzt. Da die Masse sehr schaumig war, wurden 2 g eines handelsüblichen Siliconentschäumers zugegeben.

Die Herstellung der Schmelzkleberpasten erfolgte in üblicher Weise; die Rezepturen sind in der nachfolgenden Tabelle 1 angegeben.

**Tabelle 1:**

| Rezepturen für die Schmelzkleberpasten (Angaben in Gew.-Teilen) | | | |
|---|---|---|---|
| | Nr. | | |
| | 1 | 2 | 3 |
| Dispergiermittel | 24 | 24 | 24 |
| Wasser | 41 | 41 | 41 |
| Verbindung aus Beispiel 1 | 7 | - | - |
| Verbindung aus Beispiel 2 | - | 7 | - |
| Verbindung aus Beispiel 3 | - | - | 7 |
| 5%ige Lösung eines Diurethanverdickers ^{x)} | 3 | 3 | 3 |
| 25%ige Lösung eines Na-Salzes eines Polyacrylsäureverdickers^{xx)} | 3 | 3 | 3 |
| Pulver eines thermoplastischen Polyesters ^{xxx)} | 35 | 35 | 35 |
| 1 %ige Lösung eines hochmolekularen Polyethylenglykols^{xxxx)} | 10 | 10 | 10 |

| | | | |
|---|---|---|---|
| ^{x)}= ATESYNTH ® 5112 (Dr. Th. Böhme KG) | | | |
| ^{xx)} = LATECOLL ® (BASF) neutralisiert | | | |
| ^{xxx)} = GRILTEX ® 881 (EMS-CHEMIE) | | | |
| ^{xxxx)} = MIRAPLAST ® 5147 (Dr. Th. Böhme KG) | | | |

Die Schmelzkleberpasten wurden mit einer 17 mesh Metallschablone auf ein Polyamidvlies punktförmig aufgetragen und das bedruckte Vlies 1 Minute bei 120°C in einem Heizofen mit Umluft getrocknet.

Die beschichteten Vliese wurden auf einer Durchlaufpresse mit einem Oberstoff aus 72 % Viskose und 28 % Polyestermikrofasern verpreßt. Die Temperatur in der Klebefuge betrug 120°C bzw. 110°C, die Durchlaufzeit durch die Presse 10,5 Sekunden und der Walzendruck 4 bar.

Es wurde bei der anschließenden Prüfung der Klebekraft festgestellt, daß bei beiden Temperaturen eine sehr gute Verklebung mit allen 3 Rezepturen für die Schmelzkleberpaste, erreicht wurde, was die hervorragende Eignung der erfindungsgemäßen Verbindung als Weichmacher für Kunststoffe, insbesondere in Schmelzkleberpasten, zeigte.

## Patentansprüche

1. Verbindung, die als Weichmacher für Kunststoffe geeignet ist und die Formel (1) aufweist 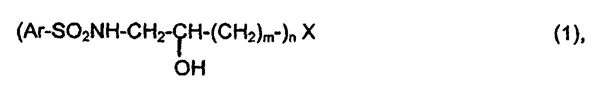 wobei
Ar für einen substituierten oder unsubstituierten Aryl-Rest,
X für einen organischen Rest,
m für eine ganze Zahl von 1 bis 4 und
n für eine ganze Zahl von 2 bis 4 steht.

2. Verbindung nach Anspruch 1, wobei der Aryl-Rest ein substituierter oder unsubstituierter Phenyl-Rest ist.

3. Verbindung nach Anspruch 1 oder 2, wobei der Rest X von einem zwei-, drei- oder vierwertigen Alkohol oder einem Polyalkylenglykol abgeleitet ist.

4. Verbindung nach Anspruch 3, wobei der von dem Polyalkylenglykol abgeleitete Rest X gemäß der Formel (2) definiert ist
O-(R¹-O)ₚ (2),
in der
R¹ für einen zweiwertigen Kohlenwasserstoff-Rest und p für eine Zahl von 1 bis 40 steht.

5. Verbindung nach Anspruch 4, wobei der zweiwertige Kohlenwasserstoff-Rest R¹ für (CH₂)₂, (CH₂)₃, (CH₂)₄ oder CH₂CH(CH₃) steht.

6. Verbindung nach Anspruch 3, wobei der von dem zweiwertigen Alkohol abgeleitete Rest X gemäß der Formel (3) definiert ist
O-R-O (3),
in der
R für einen zweiwertigen Kohlenwasserstoff-Rest -Rest steht.

7. Verbindung nach Anspruch 6, wobei der zweiwertige Kohlenwasserstoff-Rest R für (CH₂)ₒ oder (CH₂CH(CH₃))ₒ steht, wobei o eine Zahl von 1 bis 40 bedeutet.

8. Verbindung nach Anspruch 3, wobei der von dem dreiwertigen oder vierwertigen Alkohol abgeleitete Rest X für 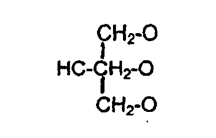 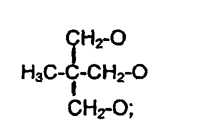 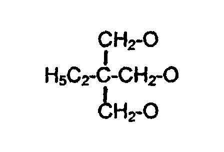 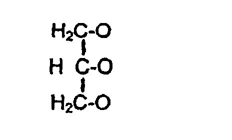 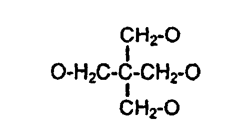 oder 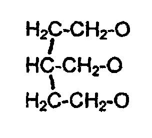 steht.

9. Verbindung nach Anspruch 1 oder 2, wobei der Rest X von einem alkoxylierten zwei-, dreioder vierwertigen Alkohol abgeleitet ist.

10. Verfahren zur Herstellung der Verbindung nach einem der vorhergehenden Ansprüche, wobei ein Epoxid der Formel (4) 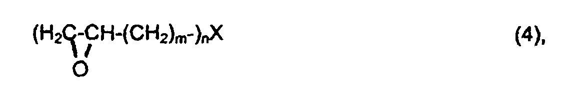 mit einem Arylsulfonamid der Formel (5)
Ar-SO₂NH₂ (5),
umgesetzt wird, wobei Ar, X, m und n wie in den vorhergehenden Ansprüchen definiert sind.

11. Verfahren nach Anspruch 10, wobei die Umsetzung in Gegenwart eines basischen Katalysators erfolgt.

12. Verwendung einer Verbindung nach einem der Ansprüche 1 bis 9 als Weichmacher für Kunststoffe.

13. Verwendung nach Anspruch 12, wobei die Kunststoffe Polymerschmelzkleber sind.

## Claims

1. Compound which is suitable for use as a plasticiser for plastics and which exhibits formula (1): 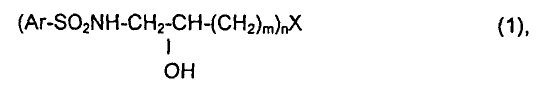 where
Ar represents a substituted or non-substituted aryl residue,
x represents an organic residue,
m represents an integer from 1 to 4 and
n represents an integer from 2 to 4.

2. Compound as per Claim 1, in which context the aryl residue is a substituted or non-substituted phenyl residue.

3. Compound as per Claims 1 or 2 in which context residue X is derived from a double, triple or quadruple valency alcohol or a polyalkalene glycol.

4. Compound as per Claim 3 in which context the residue X derived from the polyalkalene glycol is defined in accordance with formula (2)
O-(R¹-O)ₚ (2),
Where
R¹ represents a double valency hydrocarbon residue and p represents a figure from 1 to 40.

5. Compound as per Claim 4 in which context the double valency hydrocarbon residue R¹ represents (CH₂)₂, (CH₂)₃, (CH₂)₄ or CH₂CH(CH₃).

6. Compound as per Claim 3, in which context the residue X derived from the double-valency alcohol is defined in accordance with formula (3)
O-R-O (3)
Where
R represents a double valency hydrocarbon residue.

7. Compound as per Claim 6, where the double valency hydrocarbon residue R represents (CH₂)ₒ or (CH₂CH(CH₃))ₒ, in which context o means a figure from 1 to 40.

8. Compound as per Claim 3, in which context the residue X derived from the triple-valency or quadruple-valency alcohol represents 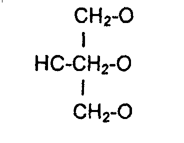 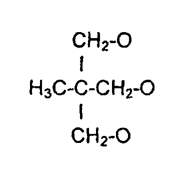 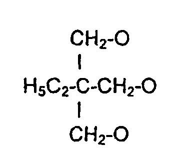 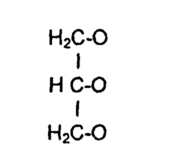 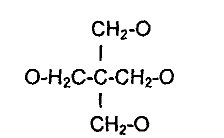 or

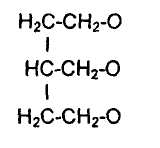

9. Compound as per Claim 1 or 2 in which context residue X is derived from an alkoxylated double, triple or quadruple valency alcohol.

10. Process for production of the compound in accordance with one of the above-mentioned claims, in which context an epoxy resin of formula (4) 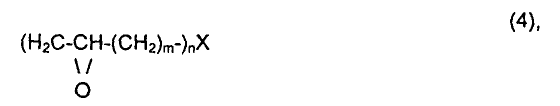 Is converted with an aryl sulphonamide of formula (5)
Ar-SO₂NH₂ (5),
Where Ar, X, m and n are defined as in the preceding Claims.

11. Process as per Claim 10, where conversion is performed in the presence of a basic catalyst.

12. Utilisation of a compound corresponding to one of Claims 1 to 9 as a plasticiser for plastics.

13. Utilisation as per Claim 12, in which context the plastics are polymeric hot-melt adhesives.

## Revendications

1. Composé approprié comme plastifiant pour plastiques et qui présente la formule (1) 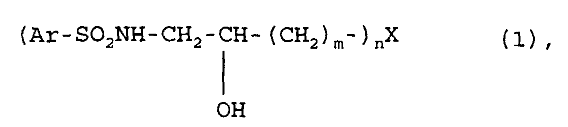 où
Ar représente un radical aryle substitué ou non substitué,
X représente un radical organique,
m représente un nombre entier entre 1 et 4 et
n représente un nombre entier entre 2 et 4.

2. Composé selon la revendication 1, où le radical aryle est un radical phényle substitué ou non substitué.

3. Composé selon la revendication 1 ou 2, où le radical X est dérivé d'un alcool di-, tri- ou tétravalent ou d'un polyalkylène glycol.

4. Composé selon la revendication 3, où le radical X dérivé du polyalkylène glycol est défini selon la formule (2)
O-(R¹-O)ₚ (2),
dans laquelle
R¹ représente un radical hydrocarbure divalent et p un nombre entre 1 et 40.

5. Composé selon la revendication 4, où le radical hydrocarbure divalent R¹ représente (CH₂)₂, (CH₂)₃, (CH₂)₄ ou CH₂CH(CH₃).

6. Composé selon la revendication 3, où le radical dérivé de l'alcool divalent X est défini selon la formule (3)
O-R-O (3),
dans laquelle
R représente un radical hydrocarbure divalent.

7. Composé selon la revendication 6, où le radical hydrocarbure divalent R représente (CH₂)ₒ ou (CH₂CH(CH₃))ₒ, où o est un nombre entre 1 et 40.

8. Composé selon la revendication 3, où le radical X dérivé de l'alcool trivalent ou tétravalent représente 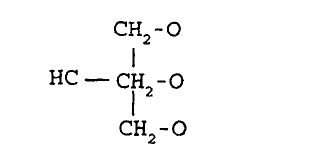 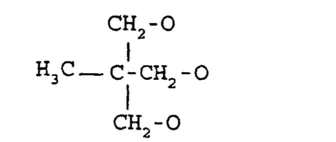 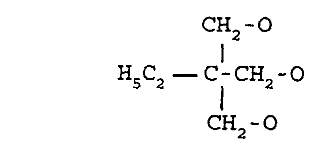 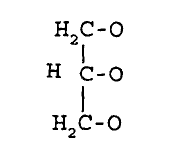 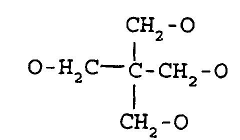 ou

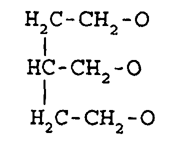

9. Composé selon la revendication 1 ou 2, où le radical X est dérivé d'un alcool di-, tri- ou tétravalent alcoxylé.

10. Procédé de préparation du composé selon l'une des revendications précédentes, où un époxyde de formule (4) 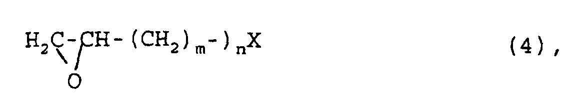 est porté à réaction avec un sulfonamide d'aryle de formule (5)
Ar-SO₂NH₂ (5),
où Ar, X, m et n sont tels que définis dans les revendications précédentes.

11. Procédé selon la revendication 10, où la réaction s'effectue en présence d'un catalyseur basique.

12. Utilisation d'un composé selon l'une des revendications 1 à 9 comme plastifiant pour plastique.

13. Utilisation selon la revendication 12, où les plastiques sont des colles polymères thermodurcissables.
